# EUROPEAN PATENT APPLICATION

(11) **EP 4 563 169 A1**
(43) Date of publication of application: **04.06.2025**
(21) Application number: 24216587.6
(22) Date of filing: 29.11.2024
(51) Int. Cl.: A61M 1/06

(54) **BREAST PUMP**

(30) Priority: 01.12.2023 CN 202323282152 U
(71) Applicant: Shenzhen Oceanwing Smart Innovations Technology Co., Ltd, Nanshan District Shenzhen Guangdong (CN)
(72) Inventor: CHEN, Liying, Shenzhen (CN); ZHANG, Zhihai, Shenzhen (CN); QIAO, Yingying, Shenzhen (CN)
(74) Representative: Bittner, Thomas L.

(57) **Abstract**

Disclosed is a breast pump comprising a horn cover, a milk bin, and a controller. The horn cover includes a cover body, and a heating device and a first connector arranged on the cover body. The first connector is electrically connected to the heating device. The controller comprises a main body, and a second connector in electrical contact with the first connector. The breast pump further comprises a sealing ring, and the milk bin includes a partition wall that comprises an electrode through hole. The sealing ring seals around a periphery of the first connector and the second connector. (Fig. 3)

## Description

The present application relates to the technical field of mother and baby products, in particular to a breast pump.

### Background

A breast pump refers to a tool used for extruding breast milk accumulated in the breast. In order to help unclog the breast, some heating breast pumps have emerged on the market. A horn cover used for contacting the breast in the heating breast pump is usually equipped with a heating element, and a main unit is configured to control the heating element, so as to achieve a hot compress function. The hot compress function is conducive to increasing the amount and speed of milk production of mothers.

However, after use, the breast pump usually requires boiling or steam disinfection, and in this process or in a process of sucking and pouring milk, moisture or milk easily enters the horn cover and the main unit, causing damage to contact points or short circuits the contact points.

### Summary

The present application describes a breast pump, which can improve connection tightness and connection stability between a horn cover and a main unit (e.g., a controller), making it difficult for liquid such as moisture or milk to enter a gap between the horn cover and the main unit, thereby reducing damage or short circuits at contact points in the horn cover or the main unit and improving the safety of the breast pump.

According to an aspect of the present application, a breast pump comprises: a cover comprising a cover body, a heating device, and a first connector, wherein the heating device and the first connector are arranged on the cover body, and the first connector is electrically connected to the heating device; a milk bin connected to the cover; and a controller comprising a main body and a second connector arranged on the main body, wherein: the milk bin comprises an electrode through hole, and the electrode through hole comprises a wall, the first connector is in electrical contact with the second connector via the electrode through hole, the cover body comprises an elastic sealing ring that wraps around a periphery of the first connector, and the wall abuts against a peripheral wall of the elastic sealing ring.

The milk bin may comprise a partition wall sandwiched between the cover body and the main body and/or the partition wall may comprise the electrode through hole.

The first connector may comprise a copper column.

At least one of a sealing rib and a sealing groove may be provided circumferentially on the peripheral wall of the elastic sealing ring. The milk bin may comprise the other one of the sealing rib and the sealing groove circumferentially on the wall and/or the sealing rib may be embedded in the sealing groove.

The elastic sealing ring may form an annular sealing portion and/or the annular sealing portion may seal around the periphery of the first connector.

The annular sealing portion may be located on the cover body, preferably to abut against the main body, and/or may surround a periphery of the second connector, preferably when the main body and the cover body are assembled to the milk bin.

The first connector may extend into the electrode through hole or pass through the electrode through hole, preferably when the main body and the cover body are assembled to the milk bin. The annular sealing portion may be located outside the electrode through hole, preferably to abut against the main body.

The milk bin may comprise a milk outlet. The milk outlet may be spaced apart from the electrode through hole along a centerline of the milk bin.

A first channel and a second channel may be connected between the milk bin and the cover and/or the electrode through hole may be located between the first channel and the second channel.

The first channel and the second channel may be symmetrically arranged on two sides of the milk outlet.

The elastic sealing ring may comprise a through hole and the first connector may be inserted into the through hole.

The elastic sealing ring may comprise at least one of a limiting groove and a limiting protrusion on a hole wall of the through hole. At least the other of the limiting groove and the limiting protrusion may be provided on a peripheral wall of the first connector. The limiting protrusion may be embedded in the limiting groove.

The first connector may comprise a fixed wrapping member and at least two first terminals. The at least two first terminals may be connected to the heating device. The fixed wrapping member may wrap a periphery of the at least two first terminals and/or may at least expose sides of the at least two first terminals facing the controller. A hardness of the fixed wrapping member may be greater than that of the elastic sealing ring.

The fixed wrapping member may wrap the periphery of the at least two first terminals by injection molding.

The main body may comprise an accommodating chamber and preferably an accommodating hole connected to the accommodating chamber. A portion of the second connector may be arranged inside the accommodating chamber and/or pass through the accommodating hole and/or extend out of the main body via the accommodating hole. The controller may comprise a sealing gasket arranged inside the accommodating chamber. The sealing gasket may wrap around a periphery of the second connector and/or may abut between two opposite walls of the accommodating chamber, preferably to seal the accommodating hole.

In an example, a breast pump comprises at least one of: a cover comprising a cover body, a heating device, and a first connector, wherein preferably the heating device and the first connector are arranged on the cover body and, more preferably, the first connector is electrically connected to the heating device; a milk bin connected to the cover; and a controller comprising a main body and a second connector arranged on the main body. Preferably, the milk bin comprises an electrode through hole and/or the electrode through hole comprises a wall and/or the first connector is in electrical contact with the second connector via the electrode through hole and/or the cover body comprises an elastic sealing ring that wraps around a periphery of the first connector and/or the wall abuts against a peripheral wall of the elastic sealing ring.

In an example, a breast pump comprises: a horn cover comprising a cover body, a heating element, and a first terminal assembly, wherein the heating element and the first terminal assembly are arranged on the cover body, and the first terminal assembly is electrically connected to the heating element; a milk bin connected to the horn cover in a sealing manner to enclose a milk storage chamber together with the horn cover; and a main unit comprising a main body and a second terminal assembly arranged on the main body, wherein the second terminal assembly is used for being in electrical contact with the first terminal assembly when the breast pump is assembled; wherein the milk bin comprises a partition wall sandwiched between the cover body and the main body, the partition wall is provided with an electrode through hole, and the electrode through hole has a hole wall; when the main body and the cover body are assembled to the milk bin, the first terminal assembly is in electrical contact with the second terminal assembly via the electrode through hole, and the cover body comprises an elastic wrapping portion, the elastic wrapping portion wraps around a periphery of the first terminal assembly, and the hole wall abuts against a peripheral wall of the elastic wrapping portion, so that the hole wall and the elastic wrapping portion limit and seal each other.

In another example, a breast pump comprises at least one of: a horn cover comprising a cover body, a heating element, and a first terminal assembly, wherein the heating element and the first terminal assembly are preferably arranged on the cover body, and the first terminal assembly is more preferably electrically connected to the heating element; a milk bin connected to the horn cover in a sealing manner, preferably to enclose a milk storage chamber together with the horn cover; and a main unit comprising a main body and a second terminal assembly arranged on the main body, wherein the second terminal assembly is preferably used for being in electrical contact with the first terminal assembly when the breast pump is assembled. Preferably, the milk bin comprises a partition wall sandwiched between the cover body and the main body and/or the partition wall is provided with an electrode through hole and/or the electrode through hole has a hole wall. More preferably, when the main body and the cover body are assembled to the milk bin, the first terminal assembly is in electrical contact with the second terminal assembly via the electrode through hole, and the cover body comprises an elastic wrapping portion, the elastic wrapping portion wraps around a periphery of the first terminal assembly, and the hole wall abuts against a peripheral wall of the elastic wrapping portion, so that the hole wall and the elastic wrapping portion limit and seal each other.

In another example, a breast pump comprises a cover (e.g., horn cover), a milk bin, and a main unit.

The horn cover comprises a cover body, a heating element, and a first terminal assembly. The heating element and the first terminal assembly are arranged on the cover body, and the first terminal assembly is electrically connected to the heating element.

The milk bin is connected to the horn cover in a sealing manner to enclose a milk storage chamber together with the horn cover.

The main unit includes a main body and a second terminal assembly arranged on the main body. The second terminal assembly is used for being in electrical contact with the first terminal assembly when the main body and the cover body are assembled.

The breast pump is provided with an annular sealing portion, the milk bin includes a partition wall sandwiched between the cover body and the main body, and the partition wall is provided with an electrode through hole. When the main body and the cover body are assembled to the milk bin, the first terminal assembly is in electrical contact with the second terminal assembly via the electrode through hole, and the annular sealing portion seals around a periphery of the first terminal assembly and the second terminal assembly.

In some examples, the annular sealing portion is located on the cover body, and the annular sealing portion surrounds the periphery of the first terminal assembly. The annular sealing portion is used for abutting against the main body and further surrounding the periphery of the second terminal assembly when the main body and the cover body are assembled to the milk bin.

In some examples, the first terminal assembly has a contact position for electrical contact with the second terminal assembly. In an electrical contact direction from the first terminal assembly to the second terminal assembly, the annular sealing portion is closer to the main body than the contact position. The first terminal assembly extends into the electrode through hole or passes through the electrode through hole when the main body and the cover body are assembled to the milk bin, and the annular sealing portion is located outside the electrode through hole to abut against the main body.

In some examples, the cover body comprises an elastic wrapping portion, the elastic wrapping portion wraps around the periphery of the first terminal assembly, and the elastic wrapping portion forms the annular sealing portion towards one end of the main body.

In some examples, one of an annular sealing rib and an annular sealing groove is provided circumferentially on a peripheral wall of the elastic wrapping portion, the partition wall is provided with the other of the annular sealing rib and the annular sealing groove circumferentially on a hole wall of the electrode through hole, and the annular sealing rib is embedded in the annular sealing groove in a sealing manner.

In some examples, the cover body comprises an elastic wrapping portion formed with a through hole; and the elastic wrapping portion wraps around the periphery of the first terminal assembly via the through hole.

In some examples, the elastic wrapping portion comprises at least one of a limiting groove and a limiting protrusion on a hole wall of the through hole, at least the other of the limiting groove and the limiting protrusion is provided on the peripheral wall of the first terminal assembly, and the limiting protrusion is embedded in the limiting groove, so that the elastic wrapping portion and the first terminal assembly limit each other in a penetration direction of the through hole; and/or the first terminal assembly is in tight fit with the elastic wrapping portion.

In some examples, the first terminal assembly includes a fixed wrapping member and at least two first terminals, the at least two first terminals are connected to the heating element, and the at least two first terminals are spaced apart; the fixed wrapping member wraps the periphery of the at least two first terminals, and at least expose the sides of the at least two first terminals facing the main unit; and the hardness of the fixed wrapping member is greater than that of the elastic wrapping portion.

In some examples, the fixed wrapping member wraps the periphery of the at least two first terminals by injection molding. The first terminals are columnar.

In some examples, the heating element is arranged inside the cover body, the through hole is in communication with the interior of the cover body, and the first terminal assembly is arranged on the heating element and extends out of the cover body from the interior of the cover body via the through hole.

In some examples, the first terminal assembly includes a first power supply positive terminal, a first temperature measurement positive terminal, and a first negative terminal spaced apart from each other, where the first power supply positive terminal and the first temperature measurement positive terminal share the first negative terminal; the second terminal assembly includes a second power supply positive terminal, a second temperature measurement positive terminal, and a second negative terminal spaced apart from each other, where the second power supply positive terminal and the second temperature measurement positive terminal share the second negative terminal; the first power supply positive terminal and the second power supply positive terminal are correspondingly in electrical contact, the first temperature measurement positive terminal and the second temperature measurement positive terminal are correspondingly in electrical contact, and the first negative terminal and the second negative terminal are correspondingly in electrical contact.

In some examples, the main body comprises an accommodating chamber and an accommodating hole in communication with the accommodating chamber, and a portion of the second terminal assembly is arranged inside the accommodating chamber, passes through the accommodating hole, and extends out of the main body via the accommodating hole. The main unit comprises a sealing gasket, and the sealing gasket is arranged inside the accommodating chamber, wraps around the periphery of the second terminal assembly, and abuts between two opposite walls of the accommodating chamber to seal the accommodating hole.

Beneficial effects of the present application include as follows: different from existing technologies, in the present application, the annular sealing portion is arranged at the connection between the first terminal assembly of the horn cover and the second terminal assembly of the main unit, and the annular sealing portion seals around the periphery of the first terminal assembly and the second terminal assembly, so that the connection between the first terminal assembly and the second terminal assembly is in a sealed environment, and liquid such as moisture or milk is unlikely to penetrate into the connection between the first terminal assembly and the second terminal assembly and further enter the interiors of the horn cover and the main unit, which can protect the conductive contact between the first terminal assembly and the second terminal assembly and can also prevent short circuits, damage, and other situations from occurring in the horn cover and the main unit, thereby improving the safety of the breast pump.

### Brief description of drawings

- FIG. 1: is a schematic diagram of an overall structure of a breast pump in an example of the present application;
- FIG. 2: is a schematic structural diagram of the breast pump shown in FIG. 1 along a section line A-A in an example;
- FIG. 3: is an enlarged schematic diagram of a partial area O of the breast pump shown in FIG. 2 in an example;
- FIG. 4: is a schematic diagram of an exploded structure of the breast pump shown in FIG. 1 in an example;
- FIG. 5: is an enlarged schematic diagram of a partial area P of the breast pump shown in FIG. 4 in an example;
- FIG. 6: is a schematic diagram of another exploded structure of the breast pump shown in FIG. 1 in an example; and
- FIG. 7: is an enlarged schematic diagram of a partial area Q of the breast pump shown in FIG. 6 in an example.

### Detailed description

The following clearly and completely describes the technical solutions in the examples of the present application in conjunction with the accompanying drawings therein. Apparently, the described examples are only some of the examples of the present application, not all of them. Based on the examples of the present application, all other examples obtained by those skilled in the art without any creative effort shall fall within the scope of protection of the present application.

A breast pump refers to a tool used for extruding breast milk accumulated in the breast. In order to help unclog the breast, some heating breast pumps have emerged on the market. A horn cover used for contacting the breast in the heating breast pump is usually equipped with a heating element, and a control main unit is configured to control the heating element, so as to achieve a hot compress function. The hot compress function is conducive to increasing the amount and speed of milk production of mothers. However, after use, the breast pump usually requires boiling or steam disinfection, and in this process or in a process of sucking and pouring milk, moisture or milk easily enters the horn cover and the control main unit, causing damage to contact points or short circuits the contact points. In order to solve the above problems, the present application provides the following examples.

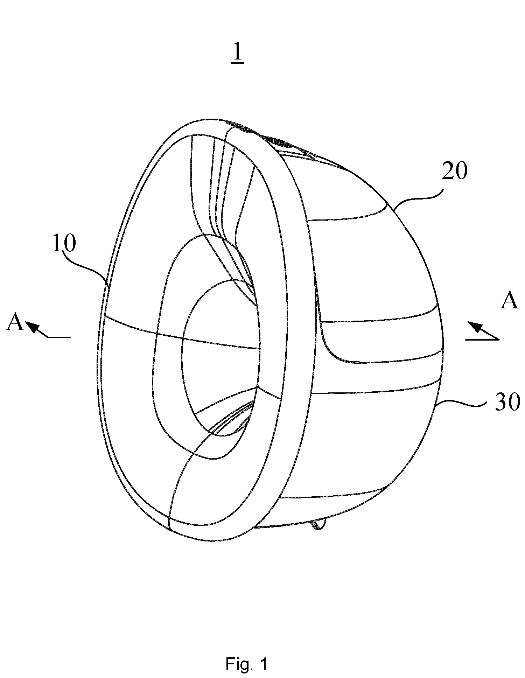

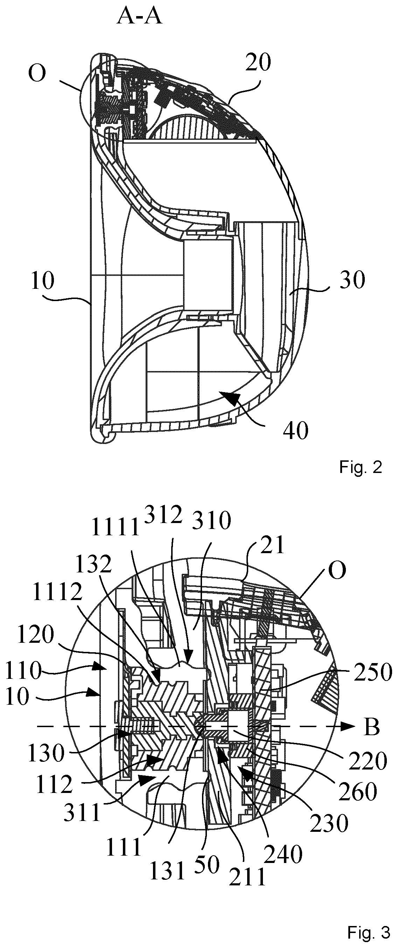

The following examples related to descriptions of a breast pump. As shown in FIG. 1 and FIG. 2, a breast pump 1 may include a horn cover 10, a milk bin 30, and a main unit 20 (e.g., a controller). The milk bin 30 may be connected to the horn cover 10 in a sealing manner to enclose a milk storage chamber 40 together with the horn cover 10.

As shown in FIG. 2 to FIG. 4, the horn cover 10 may include a cover body 110, a heating element 120 (e.g., an electric heating device, electric heat pads, warming pads), and a first terminal assembly 130 (e.g., one or more connectors). The heating element 120 and the first terminal assembly 130 may be arranged on the cover body 110, and the first terminal assembly 130 may be electrically connected to the heating element 120. The main unit 20 may include a main body 21 and a second terminal assembly 220 (e.g., one or more connectors) arranged on the main body 21, where the second terminal assembly 220 is used for being in electrical contact with the first terminal assembly 130 when the breast pump is assembled (e.g., when the main body 21 and the cover body 110 are assembled). In an example, the first terminal assembly 130 may be a first connector and second terminal assembly 220 may be a second connector.

The horn cover 10 may be used for fastening onto the nipple or mammary areola and adhering to the human breast to collect breast milk. The main unit 20 may be configured to control the horn cover 10 through the first terminal assembly 130 and the second terminal assembly 220 for negative pressure suction on the nipple, so as to stimulate the nipple for milking and collect breast milk into the milk storage chamber 40.

Further, the main unit 20 may also control the heating element 120 through the first terminal assembly 130 and the second terminal assembly 220, so that the heating element 120 can be controlled by the main unit 20 to heat the cover body 110 and then the cover body 110 heats the human body.

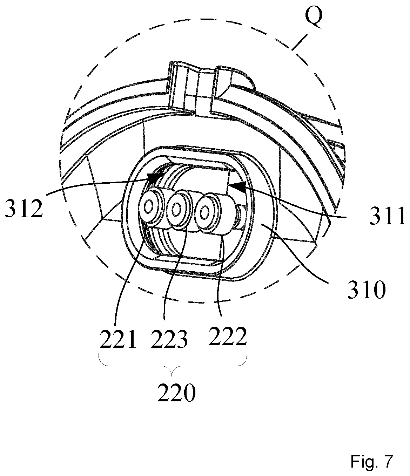

As shown in FIG. 2 to FIG. 7, the milk bin 30 may include a partition wall 310 sandwiched between the cover body 110 and the main body 21. The partition wall 310 is provided with an electrode through hole 311, and the electrode through hole 311 has a hole wall 313 (as shown in FIG. 7, the hole wall 313 protrudes towards the horn cover 10). When the main body 21 and the cover body 110 are assembled to the milk bin 30, the first terminal assembly 130 is in electrical contact with the second terminal assembly 220 via the electrode through hole 311.

The breast pump 1 may be provided with a sealing portion 50 (e.g., an annular sealing portion), and the sealing portion 50 seals around the periphery of the first terminal assembly 130 and the second terminal assembly 220.

The sealing portion 50 may be made of waterproof soft silicone, and the sealing portion 50 can prevent liquid such as moisture or breast milk from passing through. Therefore, a closed space can be formed between the annular sealing portion 50 and the main unit 20 to seal a connection position between the first terminal assembly 130 and the second terminal assembly 220.

The sealing portion 50 is arranged between the cover body 110 and the main unit 20, and the sealing portion 50 seals around the periphery of the first terminal assembly 130 and the second terminal assembly 220, so that the connection between the first terminal assembly 130 and the second terminal assembly 220 is in a sealed environment, and liquid such as moisture or milk is unlikely to penetrate into the connection between the first terminal assembly 130 and the second terminal assembly 220 and further enter the interiors of the horn cover 10 and the main unit 20, which can ensure the stability of conductive contact between the first terminal assembly 130 and the second terminal assembly 220 and can also prevent short circuits, damage, and other situations from occurring in the horn cover 10 and the main unit 20, thereby improving the safety of the breast pump 1.

In some examples, as shown in FIG. 2 to FIG. 4, the sealing portion 50 may be located on the cover body 110, and the sealing portion 50 may surround the periphery of the first terminal assembly 130. The sealing portion 50 may be used for abutting against the main body 21 and further surrounding the periphery of the second terminal assembly 220 when the main body 21 and the cover body 110 are assembled to the milk bin 30. The main body 21, the cover body 110, and the milk bin 30 may be disassembled and assembled. When the main body 21, the cover body 110, and the milk bin 30 are assembled, the main body 21 abuts against the sealing portion 50 of the cover body 110, so that the main body 21 and the sealing portion 50 can form a closed structure. Moreover, the sealing portion 50 surrounds the periphery of the first terminal assembly 130 and the second terminal assembly 220 and abuts against the main body 21. Therefore, the sealing portion 50 can seal the connection between the first terminal assembly 130 and the second terminal assembly 220, thereby improving the sealing effect of the breast pump 1.

In addition, the detachable structure can improve the assembly efficiency of the breast pump 1, facilitate the assembly and disassembly of the breast pump 1, and also facilitate the sealing structure of the cover body 110 and the sealing portion 50 at the connection between the first terminal assembly 130 and the second terminal assembly 220.

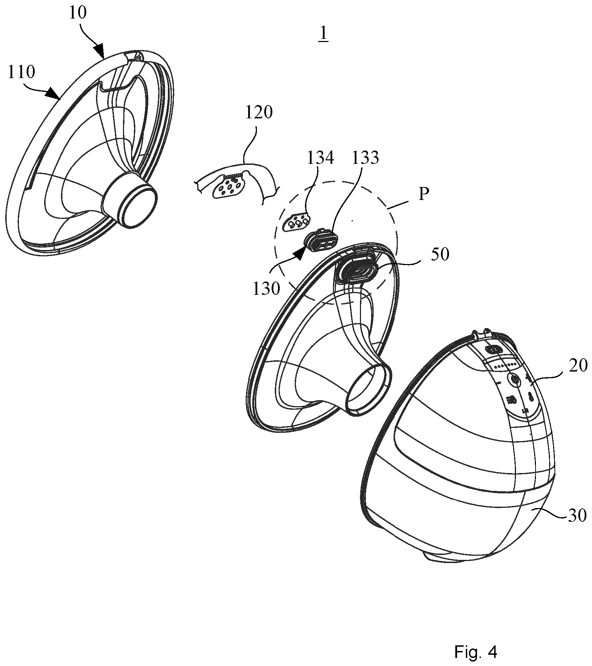

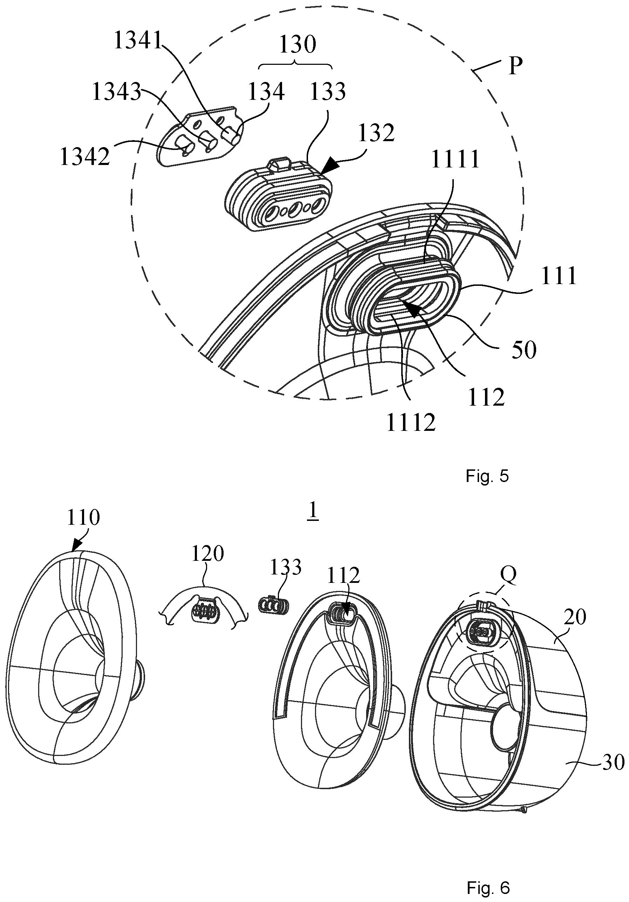

The cover body 110 may be integrally formed, and the annular sealing portion 50 may be formed on the cover body 110, so that the sealing portion 50 can be more stable. The entire cover body 110 may be made of soft silicone, and the cover body 110 may be molded by injection molding. The injection molding can simplify the formation of the cover body 110 and the sealing portion 50, and make the size of the sealing portion 50 more precise to improve the sealing effect of the breast pump 1. As shown in FIG. 4 and FIG. 6, the cover body 110 may include a first portion 1101 (e.g., a first cover) and a second portion 1102 (e.g., a second cover), and the first portion 1101 and the second portion 1102 are fastened together to form the cover body 110.

In some examples, as shown in FIG. 3, the first terminal assembly 130 may have a contact position 131 for electrical contact with the second terminal assembly 220. In an electrical contact direction from the first terminal assembly 130 to the second terminal assembly 220, the sealing portion 50 may be closer to the main body 21 than the contact position 131. The electrical contact direction from the first terminal assembly 130 to the second terminal assembly 220 may be the direction indicated by arrow B in FIG. 3.

In the electrical contact direction from the first terminal assembly 130 to the second terminal assembly 220, the sealing portion 50 may protrude compared to the contact position 131 of the first terminal assembly 130. Therefore, the sealing portion 50 may provide a margin to abut against the main body 21, then the sealing portion 50 can abut against the main body 21 more effectively during assembly, the main body 21 can press the sealing portion 50, and the pressed sealing portion 50 can compact the gap between the main unit 20 and the cover body 110, thereby forming a closed space to seal the contact position 131 of the first terminal assembly 130.

In some implementations, as shown in FIG. 3, the first terminal assembly 130 may extend into the electrode through hole 311 or pass through the electrode through hole 311 when the main body 21 and the cover body 110 are assembled to the milk bin 30, and the sealing portion 50 is located outside the electrode through hole 311 to abut against the main body 21.

The first terminal assembly 130 may be in electrical contact with the second terminal assembly 220 within the electrode through hole 311 or in electrical contact with the second terminal assembly 220 outside the electrode through hole 311. The sealing portion 50 is located outside the electrode through hole 311 and abuts against the main body 21.

In some examples, as shown in FIG. 2 to FIG. 5, the cover body 110 may include an elastic wrapping portion 111 (e.g., a silicone or rubber sealing ring or gasket), the elastic wrapping portion 111 may wrap around the periphery of the first terminal assembly 130, and the elastic wrapping portion 111 may form the sealing portion 50 towards one end of the main body 21. For example, the elastic wrapping portion 111 may be an elastic sealing ring.

For example, the elastic wrapping portion 111 may be arranged on the periphery of the first terminal assembly 130, so that the elastic wrapping portion 111 can protect the first terminal assembly 130, and when the main body 21 presses the sealing portion 50 formed by the elastic wrapping portion 111, the elastic wrapping portion 111 can seal the first terminal assembly 130, thereby preventing liquid such as moisture or milk from contacting the first terminal assembly 130 to damage the first terminal assembly 130.

As shown in FIG. 3 to FIG. 7, the hole wall 313 on the partition wall 310 may abut against a peripheral wall of the elastic wrapping portion 111, so that the hole wall 313 on the partition wall 310 and the elastic wrapping portion 111 limit and seal each other, thereby improving the structural stability and tightness of the breast pump 1.

In some implementations, as shown in FIG. 3 to FIG. 7, one of an annular sealing rib 1111 and an annular sealing groove 312 is provided circumferentially on the peripheral wall of the elastic wrapping portion 111, the partition wall 310 is provided with the other of the annular sealing rib 1111 and the annular sealing groove 312 circumferentially on the hole wall 313 of the electrode through hole 311, and the annular sealing rib 1111 is embedded in the annular sealing groove 312 in a sealing manner.

For example, the annular sealing rib 1111 may be provided on the peripheral wall of the elastic wrapping portion 111, the partition wall 310 is provided with the annular sealing groove 312 circumferentially on the hole wall 313 of the electrode through hole 311, and the annular sealing rib 1111 and the annular sealing groove 312 cooperate and buckle with each other, so that the elastic wrapping portion 111 and the partition wall 310 limit each other to achieve fixed connection, and the elastic wrapping portion 111 and the partition wall 310 are unlikely to detach from each other in a forming direction of the electrode through hole 311. The forming direction of the electrode through hole 311 may be consistent with the electrical contact direction from the first terminal assembly 130 to the second terminal assembly 220.

Moreover, the elastic wrapping portion 111 has certain elasticity. Therefore, when the elastic wrapping portion 111 and the hole wall 313 on the partition wall 310 cooperate and buckle with each other through the annular sealing rib 1111 and the annular sealing groove 312, the annular sealing rib 1111 may abut against the hole wall 313 on the partition wall 310 in the electrode through hole 311 to achieve sealing, making it difficult for liquid such as moisture or breast milk to enter the interiors of the horn cover 10 and the main unit 20 through the gap between the elastic wrapping portion 111 and the partition wall 310, thereby achieving dual sealing together with the sealing structure of the sealing portion 50 to improve the electrical safety of the breast pump 1.

In some examples, as shown in FIG. 2 to FIG. 7, the elastic wrapping portion 111 may be formed with a through hole 112. The elastic wrapping portion 111 is provided on the periphery of the first terminal assembly 130 via the through hole 112. In such a manner, the elastic wrapping portion 111 and the first terminal assembly 130 limit each other in the penetration direction of the through hole 112. The penetration direction of the through hole 112 may be indicated by arrow B in FIG. 3.

The orientation of the through hole 112 may be the electrical contact direction from the first terminal assembly 130 to the second terminal assembly 220. In this direction, because the elastic wrapping portion 111 forms the electrical contact position 131 higher than the first terminal assembly 130 and the second terminal assembly 220, the first terminal assembly 130 can be in contact with the second terminal assembly 220 within the through hole 112.

In such a manner, the elastic wrapping portion 111 can better protect the first terminal assembly 130, the connection between the elastic wrapping portion 111 and the first terminal assembly 130 can be tighter, and the elastic wrapping portion 111 can further limit the position of the first terminal assembly 130, making it less likely for the first terminal assembly 130 to shift, thereby ensuring the stability of connection between the first terminal assembly 130 and the second terminal assembly 220, and ensuring the stability of sealing between the first terminal assembly 130 and the second terminal assembly 220.

In some examples, as shown in FIG. 2 to FIG. 7, the heating element 120 may be arranged inside the cover body 110. Specifically, as shown in FIGs. 4 and 6, the cover body 110 and the heating element 120 are integrally processed by injection molding. During processing, the first portion 1101 is first formed, the heating element 120 is placed inside the first portion 1101, then the second portion 1102 is formed, and the first portion 1101 and the second portion 1102 are injection molded to obtain the cover body 110. The through hole 112 may be in communication with the interior of the cover body 110, and the first terminal assembly 130 may be arranged on the heating element 120 and extend out of the cover body 110 from the interior of the cover body 110 via the through hole 112.

In such a manner, when wrapping and sealing the first terminal assembly 130 in the through hole 112, the elastic wrapping portion 111 can further prevent moisture from entering the interior of the cover body 110 via the through hole 112 to damage the heating element 120 or short-circuit the heating element 120.

In some examples, the elastic wrapping portion 111 may be provided with at least one of a limiting groove 132 and a limiting protrusion 1112 on a hole wall of the through hole 112, at least the other of the limiting groove 132 and the limiting protrusion 1112 may be provided on the peripheral wall of the first terminal assembly 130, and the limiting protrusion 1112 is embedded in the limiting groove 132, so that the elastic wrapping portion 111 and the first terminal assembly 130 limit each other in the penetration direction of the through hole 112. The first terminal assembly 130 may be in tight fit with the elastic wrapping portion 111.

For example, as shown in FIG. 2 to FIG. 5, the elastic wrapping portion 111 may be provided with two circles of limiting protrusions 1112 on the hole wall of the through hole 112, two circles of limiting grooves 132 corresponding to the hole wall of the through hole 112 may be provided on the peripheral wall of the first terminal assembly 130, the first terminal assembly 130 may be in tight fit with the elastic wrapping portion 111, and the two circles of limiting protrusions 1112 are embedded in the two circles of limiting grooves 132, so that the elastic wrapping portion 111 and the first terminal assembly 130 can achieve a tight fit and limit each other.

Alternatively, in another implementation, the elastic wrapping portion 111 may be provided with both a limiting groove 132 and a limiting protrusion 1112 on the hole wall of the through hole 112, a limiting protrusion 1112 and a limiting groove 132 corresponding to the hole wall of the through hole 112 may also be provided on the peripheral wall of the first terminal assembly 130, and the limiting grooves 132 and the limiting protrusions 1112 on the two can match one to one, thereby achieving multiple limiting.

Through the above settings, the tightness and stability of connection between the elastic wrapping portion 111 and the first terminal assembly 130 can be improved, the elastic wrapping portion 111 and the first terminal 134 are unlikely to detach from each other in the penetration direction of the through hole 112, and the sealing effect of the elastic wrapping portion 111 can be further improved.

In some examples, as shown in FIG. 3, the main body 21 may be provided with an accommodating chamber 230 and an accommodating hole 240 in communication with the accommodating chamber 230, and a portion of the second terminal assembly 220 is arranged inside the accommodating chamber 230, passes through the accommodating hole 240, and extends out of the main body 21 via the accommodating hole 240.

As shown in FIG. 3, the accommodating chamber 230 of the main body 21 may accommodate other control elements, for example, the accommodating chamber 230 of the main body 21 may accommodate a circuit board 250, and the circuit board 250 may include processing elements such as a processor.

The accommodating hole 240 may be formed on a shell 211 of the main body 21 in a direction where the circuit board 250 faces the cover body 110, and the accommodating chamber 230 is between the circuit board 250 and the shell 211 of the main body 21. One end of the second terminal assembly 220 is electrically connected to the circuit board 250 inside the accommodating chamber 230, and the other end extends towards the accommodating hole 240, passes through the accommodating hole 240 and is in electrical contact with the first terminal assembly 130. The circuit board 250 is then electrically connected to the heating element 120 through the second terminal assembly 220 and the first terminal assembly 130 in contact with the second terminal assembly 220, and the heating element 120 can be controlled to heat.

In some implementations, as shown in FIG. 3, the main unit 20 may include a sealing gasket 260, and the sealing gasket 260 may be arranged inside the accommodating chamber 230, wraps around the periphery of the second terminal assembly 220, and abuts between two opposite walls of the accommodating chamber 230 to seal the accommodating hole 240.

The sealing gasket 260 is tightly attached to the periphery of the second terminal assembly 220, and in the extension direction of the second terminal assembly 220, one end of the sealing gasket 260 abuts against a wall surface of the accommodating chamber 230 facing the accommodating hole 240 (e.g., abuts against the circuit board 250, and the other end abuts against the chamber wall of the accommodating chamber 230 to seal the accommodating hole 240).

In such a manner, the sealing gasket 260 can seal the accommodating hole 240 at the end of the accommodating hole 240 close to the circuit board 250, thereby preventing liquid such as moisture or breast milk from entering the accommodating chamber 230 through the accommodating hole 240 to damage the circuit board 250 and other circuit elements as much as possible.

In the electrical contact direction from the first terminal assembly 130 to the second terminal assembly 220, the projection of the sealing gasket 260 may be located within the projection of the sealing portion 50, so that the sealing gasket 260 is misaligned with the sealing portion 50. Moreover, in the direction from the second terminal assembly 220 to the first terminal assembly 130, the sealing gasket 260 does not exceed the second terminal assembly 220 and the contact position 131 of the first terminal assembly 130, so that the sealing portion 50 can directly abut against the shell 211 of the main body 21, and the sealing portion 50 and the sealing gasket 260 can doubly seal the interior of the main unit 20.

In some examples, as shown in FIG. 2 to FIG. 6, the first terminal assembly 130 may include a fixed wrapping member 133 (e.g., a plastic, silicone, or rubber tube, sleeve, or gasket, plastic) and at least two first terminals 134, the at least two first terminals 134 are connected to the heating element 120, and the at least two first terminals 134 are spaced apart. The fixed wrapping member 133 may wrap the periphery of the at least two first terminals 134, and at least expose the sides of the at least two first terminals 134 facing the main unit 20.

The at least two first terminals 134 can be wrapped by the fixed wrapping member 133, and the fixed wrapping member 133 limits the at least two first terminals 134, so that the structure of the first terminal assembly 130 is more fixed. In addition, in the penetration direction of the through hole 112, the heating element 120 is connected to the at least two first terminals 134 at one end, and the other end of the heating element is in electrical contact with the second terminal assembly 220.

Moreover, at least one of a limiting groove 132 and a limiting protrusion 1112 may be provided on the fixed wrapping member 133, and correspondingly fits at least the other of the limiting groove 132 and the limiting protrusion 1112 provided on the peripheral wall of the first terminal assembly 130, thereby achieving a tight fit with the elastic wrapping portion 111.

The hardness of the fixed wrapping member 133 is greater than that of the elastic wrapping portion 111. In such a manner, the fixed wrapping member 133 can further support the elastic wrapping portion 111 and fix the position of the elastic wrapping portion 111, so that the elastic wrapping portion 111 is less likely to drive the fixed wrapping member 133 to deform and detach from each other, and the elastic wrapping portion 111 can be assembled and fastened with the fixed wrapping portion 133.

In some examples, the fixed wrapping member 133 wraps around the periphery of the at least two first terminals 134 by injection molding. Because the injection molding has the advantages of fast molding speed and precise product size, the injection molding can enable the fixed wrapping member 133 to achieve a tight fit with the at least two first terminals 134, thereby improving the structural tightness of the first terminal assembly 130.

In some implementations, the first terminals 134 are columnar. By the columnar setting, the fixed wrapping member 133 can tightly wrap the periphery of the first terminals 134 without any gap.

The first terminals 134 may all be solid copper columns. The solid copper columns are used to connect the second terminal assembly 220 of the main unit 20 and the heating element 120, so that moisture is unlikely to directly contact the heating element 120 or enter the interior of the main unit 20, which can further protect the breast pump 1.

In some implementations, as shown in FIG. 4 to FIG. 7, the first terminal assembly 130 may include a first power supply positive terminal 1341, a first temperature measurement positive terminal 1342, and a first negative terminal 1343 spaced apart from each other, where the first power supply positive terminal 1341 and the first temperature measurement positive terminal 1342 share the first negative terminal 1343. The second terminal assembly 220 may include a second power supply positive terminal 221, a second temperature measurement positive terminal 222, and a second negative terminal 223 spaced apart from each other, where the second power supply positive terminal 221 and the second temperature measurement positive terminal 222 share the second negative terminal 223.

The first power supply positive terminal 1341 and the second power supply positive terminal 221 are correspondingly in electrical contact, the first temperature measurement positive terminal 1342 and the second temperature measurement positive terminal 222 are correspondingly in electrical contact, and the first negative terminal 1343 and the second negative terminal 223 are correspondingly in electrical contact.

The first power supply positive terminal 1341, the first temperature measurement positive terminal 1342, and the first negative terminal 1343 may all be copper columns. The second power supply positive terminal 221, the second temperature measurement positive terminal 222, and the second negative terminal 223 may be probes (pogo pins) of the main unit 20.

For example, under the support of the second terminal assembly 220, the main body 21 may cooperate with the first negative terminal 1343 through the first power supply positive terminal 1341 to heat the heating element 120, so that the temperature of the heating element 120 can gradually increase. Under the support of the second terminal assembly 220, the main body 21 may cooperate with the temperature measurement positive terminal 1342 through the first power supply positive terminal 1341 to measure the temperature of the heating element 120, so as to obtain a real-time temperature of the heating element 120.

The first power supply positive terminal 1341 and the first temperature measurement positive terminal 1342 share the first negative terminal 1343, which can reduce the setting of one negative terminal, thereby reducing the space occupied by the first terminal assembly 130 and the second terminal assembly 220, simplifying the structure of the breast pump 1, then reducing the difficulty in structural design of the breast pump 1, and making the appearance of the breast pump 1 more attractive.

In the present application, the sealing portion 50 may be arranged at the connection between the first terminal assembly 130 of the horn cover 10 and the second terminal assembly 220 of the main unit 20, and the sealing portion 50 may seal around the periphery of the first terminal assembly 130 and/or the second terminal assembly 220, so that the connection between the first terminal assembly 130 and the second terminal assembly 220 is in a sealed environment, and liquid such as moisture or milk is unlikely to penetrate into the connection between the first terminal assembly 130 and the second terminal assembly 220 and further enter the interiors of the horn cover 10 and the main unit 20. This can protect the conductive contact between the first terminal assembly 130 and the second terminal assembly 220 and can also prevent short circuits, damage, and other situations from occurring in the horn cover 10 and the main unit 20, thereby improving the safety of the breast pump 1.

In one example of the present application, as shown in FIG. 6 and FIG. 7, a milk outlet 314 is provided on the partition wall 310, and the milk outlet 314 is used for connecting the milk storage chamber 40 with an external space. As shown in FIG. 6 and FIG. 7, the milk outlet 314 is arranged at an edge of the partition wall 310. For example, the milk outlet 314 may be located at a top of the partition wall 310, and the milk outlet 314 may be located on a central axis of the milk bin 30. As shown in FIG. 5, a notch 1103 is provided on the horn cover 10, the notch 1103 corresponds to the milk outlet 314, and the milk outlet 314 is surrounded by the notch 1103.

In one example of the present application, as shown in FIG. 6, the milk outlet 314 is spaced apart from the electrode through hole 311 along a centerline of the milk bin 30, and in the direction of the centerline of the milk bin 30, the milk outlet 314 at least partially overlaps with the electrode through hole 311. A first channel 315 and a second channel 316 are provided on the partition wall 310 at positions between the partition wall 310 and the horn cover 10, where the first channel 315 and the second channel 316 are both located on the side surface of the partition wall 310 facing the horn cover 10.

The first channel 315 and the second channel 316 may be of groove-shaped structures for increasing the cross-sectional area of the milk storage chamber 40 towards the milk outlet 314, so that the milk in the milk storage chamber 40 can quickly flow towards the milk outlet 314. In an example of the present application, the electrode through hole 311 is located between the first channel 315 and the second channel 316. The first channel 315 and the second channel 316 may be symmetrically arranged on two sides of the milk outlet 314. As shown in FIG. 6, the first channel 315 and the second channel 316 are symmetrically arranged on the left and right sides of the milk outlet 314 in the vertical direction of the figure or in the centerline direction of the milk bin 30.

Described above are only the examples of the present application, and the patent scope of the patent application is not limited thereto. Any equivalent structure or equivalent process transformation made using the description and accompanying drawings of the present application, directly or indirectly applied in other related technical fields, is also included in the scope of patent protection of the present application.

Hereinafter, various characteristics will be highlighted in a set of numbered clauses or paragraphs. These characteristics are not to be interpreted as being limiting on the invention or inventive concept, but are provided merely as a highlighting of some characteristics as described herein, without suggesting a particular order of importance or relevancy of such characteristics.

Clause 1: A breast pump, comprising: a cover comprising a cover body, a heating device, and a first connector, wherein the heating device and the first connector are arranged on the cover body, and the first connector is electrically connected to the heating device; and a milk bin connected to the cover.

Clause 2: The breast pump of clause 1, further comprising a controller comprising a main body and a second connector arranged on the main body, wherein: the milk bin comprises an electrode through hole, and the electrode through hole comprises a wall, the first connector is in electrical contact with the second connector via the electrode through hole, the cover body comprises an elastic sealing ring that wraps around a periphery of the first connector, and the wall abuts against a peripheral wall of the elastic sealing ring.

Clause 3: The breast pump of any one of clauses 1-2, wherein: the milk bin comprises a partition wall sandwiched between the cover body and the main body, and the partition wall comprises the electrode through hole.

Clause 4: The breast pump of any one of clauses 1-3, wherein the first connector comprises a copper column.

Clause 5: The breast pump of any one of clauses 1-4, wherein: one of a sealing rib and a sealing groove is provided circumferentially on the peripheral wall of the elastic sealing ring, the milk bin comprises the other one of the sealing rib and the sealing groove circumferentially on the wall, and the sealing rib is embedded in the sealing groove.

Clause 6: The breast pump of clause 5, wherein the elastic sealing ring forms an annular sealing portion, and the annular sealing portion seals around the periphery of the first connector.

Clause 7: The breast pump of clause 6, wherein the annular sealing portion is located on the cover body to abut against the main body and surrounds a periphery of the second connector when the main body and the cover body are assembled to the milk bin.

Clause 8: The breast pump of clause 6, wherein: the first connector extends into the electrode through hole or passes through the electrode through hole when the main body and the cover body are assembled to the milk bin, and the annular sealing portion is located outside the electrode through hole to abut against the main body.

Clause 9: The breast pump of any one of clauses 1-8, wherein: the milk bin comprises a milk outlet, and the milk outlet is spaced apart from the electrode through hole along a centerline of the milk bin.

Clause 10: The breast pump of clause 9, wherein: a first channel and a second channel are connected between the milk bin and the cover, and the electrode through hole is located between the first channel and the second channel.

Clause 11: The breast pump of clause 10, wherein the first channel and the second channel are symmetrically arranged on two sides of the milk outlet.

Clause 12: The breast pump of any one of clauses 1-11, wherein: the elastic sealing ring comprises a through hole, and the first connector is inserted into the through hole.

Clause 13: The breast pump of clause 12, wherein: the elastic sealing ring comprises at least one of a limiting groove and a limiting protrusion on a hole wall of the through hole, at least the other of the limiting groove and the limiting protrusion is provided on a peripheral wall of the first connector, and the limiting protrusion is embedded in the limiting groove.

Clause 14: The breast pump of clause 12, wherein: the first connector comprises a fixed wrapping member and at least two first terminals, the at least two first terminals are connected to the heating device, the fixed wrapping member wraps a periphery of the at least two first terminals, and at least expose sides of the at least two first terminals facing the controller, and a hardness of the fixed wrapping member is greater than that of the elastic sealing ring.

Clause 15: The breast pump of clause 14, wherein the fixed wrapping member wraps the periphery of the at least two first terminals by injection molding.

Clause 16: The breast pump of any one of clauses 1-15, wherein: the main body comprises an accommodating chamber and an accommodating hole connected to the accommodating chamber, a portion of the second connector is arranged inside the accommodating chamber, passes through the accommodating hole, and extends out of the main body via the accommodating hole, the controller comprises a sealing gasket arranged inside the accommodating chamber, and the sealing gasket wraps around a periphery of the second connector, and abuts between two opposite walls of the accommodating chamber to seal the accommodating hole.

Clause 17: A breast pump, comprising: a cover comprising a cover body and a first connector, wherein the first connector is arranged on the cover body; a milk bin connected to the cover; and a controller comprising a second connector, wherein: the first connector is in electrical contact with the second connector via an electrode through hole, and the cover body comprises a sealing ring that covers a periphery of the first connector.

Clause 18: The breast pump of clause 17, wherein the sealing ring covers a periphery of the second connector.

## Claims

1. A breast pump, comprising:
- a cover comprising a cover body, a heating device, and a first connector, wherein the heating device and the first connector are arranged on the cover body, and the first connector is electrically connected to the heating device;
- a milk bin connected to the cover; and
- a controller comprising a main body and a second connector arranged on the main body, wherein:
- the milk bin comprises an electrode through hole, and the electrode through hole comprises a wall,
- the first connector is in electrical contact with the second connector via the electrode through hole,
- the cover body comprises an elastic sealing ring that wraps around a periphery of the first connector, and
- the wall abuts against a peripheral wall of the elastic sealing ring.

2. The breast pump according to claim 1, wherein:
- the milk bin comprises a partition wall sandwiched between the cover body and the main body, and
- the partition wall comprises the electrode through hole.

3. The breast pump according to claim 1 or 2, wherein the first connector comprises a copper column.

4. The breast pump according to any of the preceding claims, wherein:
- one of a sealing rib and a sealing groove is provided circumferentially on the peripheral wall of the elastic sealing ring,
- the milk bin comprises the other one of the sealing rib and the sealing groove circumferentially on the wall, and
the sealing rib is embedded in the sealing groove.

5. The breast pump according to claim 4, wherein the elastic sealing ring forms an annular sealing portion, and the annular sealing portion seals around the periphery of the first connector.

6. The breast pump according to claim 5, wherein the annular sealing portion is located on the cover body to abut against the main body and surrounds a periphery of the second connector when the main body and the cover body are assembled to the milk bin.

7. The breast pump according to claim 5 or 6, wherein:
- the first connector extends into the electrode through hole or passes through the electrode through hole when the main body and the cover body are assembled to the milk bin, and
- the annular sealing portion is located outside the electrode through hole to abut against the main body.

8. The breast pump according to any of the preceding claims, wherein:
- the milk bin comprises a milk outlet, and
- the milk outlet is spaced apart from the electrode through hole along a centerline of the milk bin.

9. The breast pump according to claim 8, wherein:
- a first channel and a second channel are connected between the milk bin and the cover, and
- the electrode through hole is located between the first channel and the second channel.

10. The breast pump according to claim 9, wherein the first channel and the second channel are symmetrically arranged on two sides of the milk outlet.

11. The breast pump according to any of the preceding claims, wherein:
- the elastic sealing ring comprises a through hole, and
- the first connector is inserted into the through hole.

12. The breast pump according to claim 11, wherein:
- the elastic sealing ring comprises at least one of a limiting groove and a limiting protrusion on a hole wall of the through hole,
- at least the other of the limiting groove and the limiting protrusion is provided on a peripheral wall of the first connector, and
- the limiting protrusion is embedded in the limiting groove.

13. The breast pump according to claim 11 or 12, wherein:
- the first connector comprises a fixed wrapping member and at least two first terminals,
- the at least two first terminals are connected to the heating device,
- the fixed wrapping member wraps a periphery of the at least two first terminals, and at least expose sides of the at least two first terminals facing the controller, and
- a hardness of the fixed wrapping member is greater than that of the elastic sealing ring.

14. The breast pump according to claim 13, wherein the fixed wrapping member wraps the periphery of the at least two first terminals by injection molding.

15. The breast pump according to any of the preceding claims, wherein:
- the main body comprises an accommodating chamber and an accommodating hole connected to the accommodating chamber,
- a portion of the second connector is arranged inside the accommodating chamber, passes through the accommodating hole, and extends out of the main body via the accommodating hole,
- the controller comprises a sealing gasket arranged inside the accommodating chamber, and
- the sealing gasket wraps around a periphery of the second connector, and abuts between two opposite walls of the accommodating chamber to seal the accommodating hole.
